(19) Europäisches Patentamt
European Patent Office
Office européen des brevets

(11) Veröffentlichungsnummer: **0 558 816 B1**

(12) **EUROPÄISCHE PATENTSCHRIFT**

(45) Veröffentlichungstag der Patentschrift: **11.10.95**

(51) Int. Cl.$^{6}$: **C07C 15/16**, C07C 2/86, C07C 7/148

(21) Anmeldenummer: **92121728.7**

(22) Anmeldetag: **19.12.92**

# (54) Verfahren zur Herstellung von monobenzylierten Alkylbiphenylen und deren Verwendung.

(30) Priorität: **22.02.92 DE 4205504**
**11.09.92 DE 4230514**

(43) Veröffentlichungstag der Anmeldung:
**08.09.93 Patentblatt 93/36**

(45) Bekanntmachung des Hinweises auf die Patenterteilung:
**11.10.95 Patentblatt 95/41**

(84) Benannte Vertragsstaaten:
**BE DE FR GB IT NL**

(56) Entgegenhaltungen:
**EP-A- 0 435 737**
**DE-A- 3 940 331**

(73) Patentinhaber: **HÜLS AKTIENGESELLSCHAFT**

**D-45764 Marl (DE)**

(72) Erfinder: **Hons, Gerd, Dr.**
**Dorstener Strasse 445**
**W-4690 Herne 2 (DE)**
Erfinder: **Wachholz, Gerhard, Dr.**
**Lipper Weg 193**
**W-4370 Marl (DE)**
Erfinder: **Voges, Heinz-Werner, Dr.**
**Im Gorden 45**
**W-4270 Dorsten (DE)**

EP 0 558 816 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von monobenzylierten Alkylbiphenylen der allgemeinen Formel I

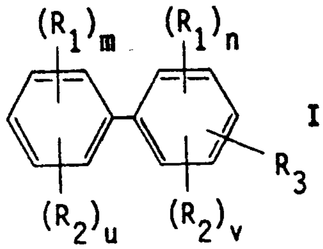

I

worin $R_1$ = Methyl, $R_2$ = Ethyl oder Isopropyl, $R_3$ = Benzyl,
m, n, u und v = 0 - 2
m + n ≦ 4, u + v ≦ 2 sowie
0 < (n + m) + (u + v) ≦ 4,
durch Benzylierung von Alkylbiphenylen der Formel II, worin

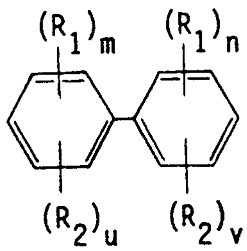

II

$R_1$, $R_2$, $R_3$ sowie m, n, u und v die gleiche Bedeutung wie in Formel I haben;
und Entfernung von chlorhaltigen Nebenprodukten durch Behandlung derselben mit metallischem Natrium sowie deren Verwendung als Wärmeträger.

Die monobenzylierten Alkylbiphenyle der Formel I und ihre Gemische sind Flüssigkeiten tiefen Stockpunktes (< 0 °C) und hohen Siedepunktes (> 300 °C), die sich durch besonders hohe thermische Beständigkeit auszeichnen und daher z. B. als Wärmeübertragungsöle verwendet werden können.

Ausgangsstoffe zur Herstellung der Verbindungen der Formel I sind Biphenyl, Methylbiphenyle oder auch die technischen Gemische von Biphenyl und Methylbiphenylen, die aus in der erdölverarbeitenden Industrie anfallenden Destillationsrückständen der Toluoldealkylierungsanlagen gewonnen werden können.

Geht man von Methylbiphenylen aus, so gelangt man durch das erfindungsgemäße Benzylierungsverfahren in einer Reaktionsstufe direkt zu den Verbindungen der Formel I mit u + v = 0.

Geht man von Biphenyl oder den besagten Biphenyl-/Methylbiphenyl-Gemischen aus, so wird die Synthese der Zielprodukte der Formel I in zwei Stufen durchgeführt:

1. Ethylierung von Biphenyl bzw. Biphenyl-/Methylbiphenyl-Gemisch zu dem Vorprodukt der Formel II und
2. dessen erfindungsgemäße Monobenzylierung zu I.

Die Ethylierung von Biphenyl bzw. Biphenyl-/Methylbiphenyl-Gemischen kann in Analogie zu der bekannten Ethylierung des Benzols z. B. durch Umsetzung mit Ethylen (und/oder Ethylchlorid) in Gegenwart von Friedel-Crafts-Katalysatorenwie $AlCl_3$, $BF_3$, $FeCl_3$, $H_3PO_4$ in flüssiger Phase durchgeführt werden. Ein Verfahren, das mit $AlCl_3$ arbeitet, wurde bereits in US-PS 2 172 391 beschrieben. Es kann aber auch die aus der technischen Herstellung von Ethylbenzol bekannte Transalkylierung zur Herstellung von Ethylbiphenyl herangezogen werden. Dazu läßt man "Polyethylbenzole" - das sind Destillationsfraktionen bestehend aus (isomeren) Di- und Triethylbenzolen, so wie sie als Nebenprodukte in einer Ethylbenzolfabrik anfallen - mit Biphenyl oder Biphenyl-/Methylbiphenyl-Gemischen unter der Wirkung eines Friedel-Crafts-Katalysators, wie z. B. $AlCl_3$, in der Flüssigphase reagieren. Dabei findet eine Übertragung von Ethylgruppen vom "Polyethylbenzol" auf Biphenyl/Methylbiphenyl unter Bildung von Ethylbiphenylen/Ethylmethylbiphenylen statt.

Die Herstellung von Ethylbiphenylen durch Transalkylierung mit "Polyethylbenzol" ist in einigen Patentschriften beschrieben, so z. B. in JA-OS 3 041-184, US-PS 4 943 553 und JA-PS 62 252 733. Ausgearbeitete Vorschriften zur Durchführung der Transalkylierung sind als nicht erfindungsgemäße Beispiele A und B weiter unten mit aufgeführt.

Methylbiphenyle, Ethylbiphenyle sowie die Gemische aus Ethyl- und Methylethylbiphenylen können in bekannter Weise durch Reaktion mit Benzylchlorid unter der Wirkung von Friedel-Crafts-Katalysatoren, wie $AlCl_3$, benzyliert werden, wie z. B. in der DE-OS 31 27 970 erwähnt wird. Diese gebräuchliche Verfahrensweise hat aber zum einen den Nachteil, daß im Anschluß an die Benzylierungsreaktion eine Behandlung des Reaktionsgemisches mit Wasser oder wäßriger Alkalilauge folgen muß, um den Katalysator zu zerstören und auszuwaschen. Die Entsorgung der dabei anfallenden, die Katalysatorreste enthaltenden wäßrigen Phasen wirft unter den heute geltenden Abwasser- und Abfallvorschriften Probleme auf. Zum anderen enthalten die vom $AlCl_3$-Katalysator befreiten Reaktionsgemische erfahrungsgemäß chlorhaltige organische Nebenprodukte meist unbekannter Konstitution. Der Chlorgehalt des Reaktionsgemisches beträgt gewöhnlich 500-1 500 ppm.

In EP-A-0 435 737 werden chlorhaltige organische Nebenprodukte bei der Herstellung von Oligomeren des Benzyltoluols mit Hilfe von Natrium, vorzugsweise jedoch mit Hilfe von Natriummethylat abgetrennt.

Die Abwasserentsorgungsprobleme können vermieden werden, wenn man die Umsetzung mit Benzylchlorid in Gegenwart katalytisch wirksamer saurer Tonmineralien vom Typ des Montmorillonits durchführt, die man nach Beendigung der Reaktion durch Filtration vom Reaktionsgemisch abtrennen kann.

Die Benzylierung von nicht-substituiertem Biphenyl unter der katalytischen Wirkung eines Montmorillonits in Pulverform ist in der DE-OS 3 940 331 (= EP-A 0 431 265) beschrieben. Dort ist als Ziel die Herstellung von Reaktionsgemischen genannt, die reich an in 4-Stellung substituiertem Benzyl-biphenyl sind. Der Feststoffkatalysator wird in einer Menge von 2,5-50 Gew.-% und - wie den Beispielen zu entnehmen ist - bevorzugt in einer Menge von 20 Gew.-%, bezogen auf eingesetztes Biphenyl, angewendet. Kennzeichnend für die Arbeitsweise der DE-OS 3 940 331 ist ferner, daß das Gemisch der Reaktionspartner Biphenyl und Benzylchlorid mit dem pulverförmigen Katalysator versetzt wird und sodann 2 Stunden bei 100 °C gerührt wird.

Diese Verfahrensweise, angewendet auf Methylbiphenyle, Ethylbiphenyle und Gemische aus Ethyl- und Methylethylbiphenylen anstelle von Biphenyl, führt zu einer heftigen, hinsichtlich der einzuhaltenden optimalen Temperatur und der HCl-Gasentwicklung nur schwierig zu kontrollierenden Reaktion. Ein schwerer wiegender Nachteil dieser Verfahrensweise ist jedoch, daß die Ausbeute an dem als Wärmeträger brauchbaren monobenzylierten Reaktionsgemisch I vermindert ist, da sich zahlreiche Nebenprodukte bilden, darunter solche, die im Biphenylkern zwei oder mehr Benzylreste tragen wie solche, deren Benzylrest $R_3$ benzyliert ist. Zudem stellt man fest, daß Reaktionsgemische, die bei dieser nur schwer kontrollierbaren Reaktionsführung erhalten werden, wiederum chlorhaltige organische Nebenprodukte enthalten, die den Gesamt-Chlorgehalt der Reaktionsmischung auf 500-1 500 ppm stellen. Diese chlorhaltigen Verbindungen lassen sich gewöhnlich nicht durch Destillation abtrennen, sondern schleppen sich in die Destillationsfraktion, so auch in die gewünschten Fraktionen der monobenzylierten Alkylbiphenyle fort. Den Einsatz derart hoch chlorhaltiger Produkte als Wärmeübertragungsflüssigkeiten lehnen industrielle Anwender gewöhnlich ab.

Die Aufgabe, alkylierte Biphenyle der Formel II mit Benzylchlorid unter Bildung von monobenzylierten Alkylbiphenylen der Formel I in kontrollierbarer Weise zur Umsetzung zu bringen, wird erfindungsgemäß gelöst, indem das als Katalysator wirkende Aluminiumhydrosilikat in einer Menge von 0,1 bis 1,5 Gew.-%, bevorzugt von 0,5 bis 1 Gew.-%, bezogen auf die Menge des zu benzylierenden Alkylbiphenyls unter speziellen Bedingungen eingesetzt wird.

Gegenstand der Erfindung ist ein Verfahren zur Herstellung von monobenzylierten Alkylbiphenylen der Formel I, worin $R_1$ = Methyl, $R_2$ = Ethyl oder Isopropyl, $R_3$ = Benzyl,

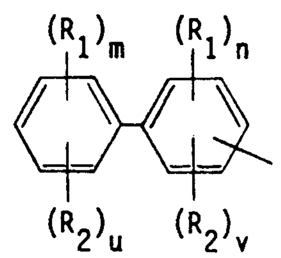
I

m, n, u und v = 0 - 2

$m + n \leqq 4$, $u + v \leqq 2$ sowie

$0 < (m + n) + (u + v) \leqq 4$

durch Umsetzung von Alkylbiphenylen der Formel II, worin $R_1$, $R_2$ sowie

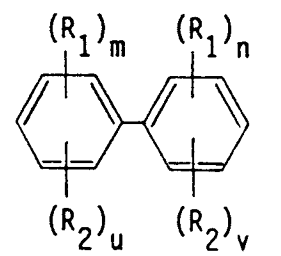
II

m, n, u, v die gleiche Bedeutung wie in Formel I haben, mit Benzylchlorid, welches dadurch gekennzeichnet ist, daß die Reaktion mit 0,2-0,7 Mol Benzylchlorid pro Mol Alkylbiphenyl der Formel II in Gegenwart von 0,1 bis 1,5 Gew.-% bezogen auf II, eines suspendierten Aluminiumhydrosilikats mit der idealisierten Formel $Al_2O_3 \cdot 4SiO_2 \cdot H_2O$ mit einer Oberfläche von 190-250 $m^2/g$ bei Temperaturen von 100 bis 140 °C im Zulaufverfahren durchgeführt wird, wobei der Zulaufzeitraum für das Benzylchlorid sich über 0,5 bis 5 Stunden erstreckt, und das Reaktionsgemisch nach Entfernung des Katalysators gegebenenfalls durch Erhitzen mit metallischem Natrium von organischen Chlorverbindungen gereinigt wird.

Der Aluminiumhydrosilikat-Katalysator, ein Mineral aus der Klasse der Montmorillonite mit der idealisierten Formel $Al_2O_3 \cdot 4SiO_2 \cdot H_2O$ wird als Pulver mit einer Oberfläche von 190 bis 250 $m^2/g$, so wie käuflich zu erwerben, in der genannten

Menge unter Rühren in dem zu benzylierenden Alkylbiphenyl suspendiert. Zu der auf 100 bis 140 °C temperierten Suspension wird sodann unter fortgesetztem Rühren die berechnete Menge Benzylchlorid, gewöhnlich 0,2 bis 0,7 Mol, bevorzugt 0,5 Mol pro Mol eingesetztem Alkylbiphenyl gleichmäßig verteilt über einen längeren Zeitraum zugegeben. Die Reaktion setzt sofort nach Beginn der Benzylchlorid-Zugabe ein, wie an der Entwicklung von Chlorwasserstoffgas erkennbar ist. Der HCl-Gasstrom wird am Kopf des Reaktionsgefäßes abgeleitet und z. B. einem Waschturm zugeführt, in dem HCl in Wasser oder Natronlauge absorbiert wird. Der Zeitraum, über den hinweg das Benzylchlorid zugegeben wird, beträgt mindestens 0,5 Stunden, besser 2 bis 5 Stunden. Man erreicht dadurch, daß die Stationärkonzentration an Benzylchlorid niedrig bleibt und demzufolge die Nebenproduktbildung zurückgedrängt wird und außerdem, daß die Einhaltung einer konstanten Reaktionstemperatur sowie die Ableitung und gegebenenfalls die Absorption des Chlorwasserstoffgases erleichtert werden. Überraschend günstig wirkt sich die erfindungsgemäße Verfahrensweise der Benzylierung insofern aus, daß der Anteil an chlorhaltigen organischen Nebenprodukten unbekannter Konstitution schon in dem vom Katalysator abfiltrierten Reaktionsgemisch im Vergleich zu der in DE-OS 3 940 331 beschriebenen Arbeitsweise drastisch vermindert ist. Im allgemeinen beträgt der Chlorgehalt im rohen Reaktionsgemisch 20 bis 50 ppm. Die anschließende fraktionierende Destillation dieses Gemisches liefert die gewünschten monobenzylierten Alkylbiphenyle mit Chlorgehalten von ca. 20 ppm, die oftmals den Qualitätsansprüchen genügen. Für höchste Ansprüche muß der Chlorgehalt noch weiter abgesenkt werden, z. B. bis zu Restgehalten von < 10 ppm.

Wie außerdem gefunden wurde, gelingt die Eliminierung des organisch gebundenen Chlors in überraschend weitgehendem Maße, indem man nach Beendigung der Benzylierungsreaktion den Katalysator abtrennt und das Reaktionsgemisch mit metallischem Natrium in einer Menge von 0,5 bis 1 Gew.-%, bezogen auf die Menge des Reaktionsgemisches, einige Stunden auf eine Temperatur von 200 bis 210 °C erhitzt. Nachdem man darauf das nicht verbrauchte Natrium durch Zugabe von überschüssigem Wasser in Natronlauge überführt Und diese mit dem gebildeten Natriumchlorid abgetrennt hat, erhält man eine organische Phase, die praktisch vollständig von organischen Chlorverbindungen befreit ist.

Die erfindungsgemäße Verfahrensweise zur Monobenzylierung von Alkylbiphenylen und zu deren Entchlorung geht im einzelnen aus den Beispielen hervor, denen - nicht erfindungsgemäße - Beschreibungen der Herstellung von Ethylbiphenylen vorangestellt werden.

Ein weiterer Gegenstand der Erfindung ist die Verwendung der nach den Ansprüchen 1 bis 5 hergestellten monobenzylierten Alkylbiphenyle als Wärmeträger.

**Beispiele (nicht erfindungsgemäß)**

A) Ethylierung von Biphenyl durch Transalkylierung

Ein Gemisch von 3 Gewichtsteilen Biphenyl und 2 Gewichtsteilen "Polyethylbenzol" (27,5 % Di- und 72,5 % Triethylbenzol) wird mit 0,5 bis 1,5 Gew.-% Aluminiumtrichlorid, bezogen auf die Menge des eingesetzten Biphenyls, versetzt und unter Rühren 3 bis 5 Stunden auf 120 °C erhitzt. Danach kühlt man auf ca. 50 °C ab und versetzt das Reaktionsgemisch unter Rühren mit Wasser. Man läßt sich die Phasen scheiden und trennt die untere wäßrige Phase, die den (hydrolysierten) Katalysator enthält, ab. Ein nochmaliges Waschen der organischen Phase mit Wasser entfernt letzte Reste von Katalysatorbestandteilen. Das Produktgemisch hat bei dem gewählten Einsatzverhältnis von Biphenyl und "Polyethylbenzol" eine Zusammensetzung in den folgenden Grenzen: Benzol 3 bis 4 Gew.-%, Mono-, Di- und Triethylbenzole zusammen 12 bis 19 Gew.-%, unumgesetztes Biphenyl 18 bis 20 Gew.-%, isomere Monoethylbiphenyle 33 bis 36 Gew.-%, isomere Diethylbiphenyle 19 bis 22 Gew.-%, isomere Triethylbiphenyle 4 bis 6 Gew.-% und sonstige Bestandteile ca. 1 Gew.-%. Dieses Gemisch wird einer fraktionierenden Destillation bei vermindertem Druck, z. B. bei 20 mbar, unterworfen. Nach dem Abdestillieren von Benzol, den Ethylbenzolen und unverändertem Biphenyl erhält man Fraktionen, die Mono- und Diethylbiphenyl in wechselnder Zusammensetzung enthalten, jedoch frei von Triethylbiphenyl sind. Ein Gemisch mit der beispielhaften Zusammensetzung 64 Gew.-% isomere Monoethylbiphenyle und 36 Gew.-% isomere Diethylbiphenyle ist ein geeignetes Einsatzprodukt für die erfindungsgemäß durchgeführte Benzylierungsreaktion.

B) Ethylierung von Biphenyl-/Methylbiphenyl-Gemisch durch Transalkylierung

39 Gew.-T. eines Gemisches von Di- und Triethylbenzol (27,5 % Di- und 72,5 % Triethylbenzol) und 61 Gew.-T. eines Gemisches von Biphenyl und (isomeren) Monomethylbiphenylen (61,7 % Biphenyl und 38,3 % Methylbiphenyle) wurden gemischt und mit 0,9 Gew.-%, bezogen auf die Gesamtmenge, an Aluminiumtrichlorid versetzt. Das Reaktionsgemisch wird unter Rühren auf 120 °C erwärmt und ca. 3 h unter fortgesetztem Rühren bei dieser Temperatur gehalten. Die gaschromatographischen Analysen von Proben, die dem Reaktionsgemisch entnommen werden, zeigen, daß nach der genannten Reaktionszeit das Transalkylierungsgleichgewicht eingestellt ist. Nun wird dem Reaktionsgemisch Wasser in einer Gewichtsmenge, die der Gewichtsmenge des Aluminiumchlorids entspricht, zugesetzt und so lange gerührt, bis der rot-farbene, gelöste Katalysatorkomplex zerstört ist, was an der Entfärbung des Reaktionsgemisches erkannt wird. Der Rührer wird abgestellt, und man läßt sich die Phasen scheiden. Nach Abtrennen der unteren wäßrigen Phase, die den hydrolysierten Katalysator enthält, wäscht man die organische Phase noch mehrmals mit Wasser nach. Die organische Phase enthält laut gaschromatographischer Analyse 3,5 % Benzol, 30,5 % Mono-, Di- und Triethylbenzol (im Verhältnis ca. 2 : 4 : 1), 32 % unverändertes Biphenyl und Methylbiphenyl (im Verhältnis ca. 1,6 : 1), 2 % Triethylbiphenyl und andere Hochsieder sowie 32 % eines durch nachfolgende Destillation gewinnbaren Gemisches aus 45 % Ethylbiphenyl, 34 % Ethyl-methyl-biphenyl, 15,5 % Diethyl-biphenyl und 5 % Diethyl-methyl-biphenyl, welches sich als Einsatzstoff für die erfindungsgemäße Benzylierungsreaktion eignet.

Beispiel 1

In 350 kg eines Gemisches aus 64 Gew.-% Monoethyl- und 36 Gew.-% Diethylbiphenyl, so wie im Beispiel A) erhalten, werden 3,5 kg eines käuflichen Montmorillonit-Katalysators in Pulverform (Herkunft: Fa. Südchemie, Bezeichnung des Katalysators: K 306) unter Rühren suspendiert. Die gerührte Suspension wird auf 140 °C erwärmt. Im Verlauf von 5 h werden unter fortgesetztem Rühren 119 kg reines Benzylchlorid zugeführt, wobei Sorge getragen wird, das sich bildende Chlorwasserstoffgas am Kopf des Reaktionsgefäßes abzuführen und in einem mit Wasser beschickten Waschturm zu absorbieren. Über die gesamte Zugabezeit des Benzylchlorids bleibt die Stationärkonzentration des Benzylchlorids im Reaktionsgemisch auf sehr niedrigem Niveau, bei ca. 0,1-0,2 Gew.-%. Nach abgeschlossener Benzylchorid-Zugabe läßt man noch ca. 1 h weiterrühren, wonach die Benzylchlorid-Konzentration unter 0,1 Gew.-% abgesunken und die HCl-Entwicklung beendigt ist. Der Reaktorinhalt wird nun auf ca. 80 °C abgekühlt. Durch Anlegen eines schwachen Vakuums entfernt man das in der Suspension gelöste Chlorwasserstoffgas, kühlt dann auf ca. 40 °C ab und filtriert den suspendierten Katalysator ab. Das Filtrat, eine nur schwach gelblich gefärbte Flüssigkeit, weist einen Chlorgehalt von 28 ppm auf. Die Destillation des Filtrats, insgesamt 344 kg, liefert 178 kg eines Gemisches aus unumgesetztem Mono- und Diethylbiphenyl zurück, die erneut in die Reaktion eingespeist werden können, sowie 249 kg eines Gemisches aus Benzyl-ethyl-biphenyl und Benzyl-di-ethyl-biphenyl (im Verhältnis 2,36 : 1). Als Destillationsrückstand verbleiben 6,5 kg. Der Chlorgehalt in dem Benzyl-ethyl-, Benzyl-diethyl-biphenylgemisch beträgt 21 ppm.

Beispiel 2

Die Benzylierung des Gemisches aus Ethyl- und Diethylbiphenyl gemäß Beispiel 1 wird wiederholt. Nach Abtrennung des Katalysators werden 400 kg des Reaktionsgemisches, das einen Chlorgehalt von 28 ppm aufweist, in einen beheizbaren Rührreaktor gegeben. Das über der Flüssigkeit befindliche Luftvolumen wird durch Einleiten von deoxygeniertem Stickstoff verdrängt. Danach fügt man 2 kg metallisches Natrium (in Stangenform) hinzu und erhitzt den Reaktorinhalt unter fortwährendem Rühren und Überleiten von Stickstoff auf 210 °C. Bei dieser Temperatur wird das Rühren 6 Stunden fortgeführt, wobei auch fortwährend Stickstoff in den Gasraum eingeleitet wird. Danach kühlt man auf ca. 50 °C ab und fügt innerhalb von 1 h, nach wie vor unter strömendem Stickstoff, 90 kg einer 15 gew.-%igen wäßrigen Natriumsulfatlösung hinzu. Der durch Reaktion des unverbrauchten Natriums mit Wasser freiwerdende Wasserstoff wird mit dem Stickstoffstrom gefahrlos aus dem Gasraum ausgetragen. Man erwärmt den Reaktorinhalt sodann auf 80 °C und läßt nach Abstellen des Rührers sich die Phasen scheiden. Die untere wäßrige Phase wird abgetrennt, die obere organische Phase noch mehrmals mit 15 %iger Natriumsulfatlösung gewaschen, bis die jeweils abgeschiedene untere wäßrige Phase neutral reagiert. Die organische Phase weist einen Gehalt an organisch gebundenem Chlor von 2 ppm auf. Die fraktionierende Destillation gemäß Beispiel 1 liefert in unveränderter Zusammensetzung ein Gemisch aus Benzyl-ethyl-biphenyl und Benzyl-diethyl-biphenyl mit einem Chlorgehalt von ≦ 2 ppm. Der Stockpunkt des Gemisches beträgt -25 °C, es beginnt bei 370 °C zu sieden. Als Wärmeübertragungsflüssigkeit ist es vorzüglich geeignet.

Beispiel 3

In 150 kg eines Gemisches aus Ethylbiphenyl (45,5 %), Ethyl-methyl-biphenyl (34 %), Diethylbiphenyl (15,5 %) und Diethyl-methyl-biphenyl (5 %) werden 0,75 kg Montmorillonit-Katalysator in Pulverform suspendiert. Die Suspension wird auf 140 °C erhitzt. Bei dieser Temperatur werden unter Rühren im Verlauf von 4 h 49,3 kg Benzylchlorid zugegeben. Man verfährt weiter, wie im Beispiel 1 beschrieben, und erhält nach Abtrennung des Katalysators ein Reaktionsgemisch mit einem Chlorgehalt von 40 ppm. Dieses wird, wie im Beispiel 2 beschrieben, einer Behandlung mit 1 kg metallischem Natrium unterworfen, wodurch der Gehalt an organisch gebundenem Chlor auf 2 ppm gesenkt wird. Die abschließende Destillation liefert als Vorlauf 79 kg des oben genannten Einsatzstoffgemisches zurück sowie 93 kg eines Gemisches aus monobenzyliertem Ethyl-, Ethyl-methyl-, Diethyl- und Diethyl-methyl-biphenyl mit einem Restchlorgehalt von ≦ 2 ppm, mit einem Stockpunkt von -26 °C und einem Siedepunkt (Siedebeginn) von 342 °C.

Beispiel 4

Die Benzylierung wird analog der Fahrweise aus Beispiel 1 mit Isopropyldiphenyl als Edukt wiederholt. Hierzu werden 17,6 kg Isopropyldiphenyl zusammen mit 0,09 kg Montmorillonit-Katalysator (Hersteller: Firma Südchemie, Bezeichnung des Katalysators: K 10) auf 140 °C erwärmt. Bei dieser Temperatur werden unter Rühren im Verlauf von 2,5 h 38,4 kg Benzylchlorid zugegeben. Man verfährt weiter wie in Versuch 1 beschrieben und erhält ein Gemisch, das nach einer Behandlung mit metallischem Natrium destilliert werden kann. Die abschließende Destillation liefert neben 13 kg unumgesetztem Isopropyldiphenyl 6,5 kg des gewünschten Produktes Benzylisopropyldiphenyl. Das Produkt hat einen Stockpunkt von -17 °C, eine kin. Viskosität bei 20 °C von 193 $mm^2/s$ und einen Siedepunkt (Siedebeginn) von 363 °C.

**Patentansprüche**

1. Verfahren zur Herstellung von monobenzylierten Alkylbiphenylen der Formel I, worin $R_1$ = Methyl, $R_2$ = Ethyl oder Isopropyl, $R_3$ = Benzyl,

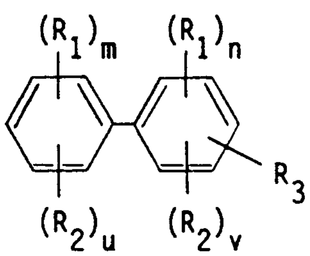

I

m, n, u und v = 0 - 2
m + n ≦ 4, u + v ≦ 2 sowie
0 < (m + n) + (u + v) ≦ 4
durch Umsetzung von Alkylbiphenylen der Formel II, worin $R_1$, $R_2$ sowie

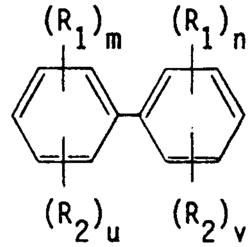

II

m, n, u, v die gleiche Bedeutung wie in Formel I haben, mit Benzylchlorid,
dadurch gekennzeichnet,
daß die Reaktion mit 0,2-0,7 Mol Benzylchlorid pro Mol Alkylbiphenyl der Formel II in Gegenwart von 0,1 bis 1,5 Gew.-% bezogen auf II, eines suspendierten Aluminium-hydrosilikats mit der idealisierten Formel $Al_2O_3 \cdot 4SiO_2 \cdot H_2O$ mit einer Oberfläche von 190-250 $m^2/g$ bei Temperaturen von 100 bis 140 °C im Zulaufverfahren durchgeführt wird, wobei der Zulaufzeitraum für das Benzylchlorid sich über 0,5 bis 5 Stunden erstreckt, und das Reaktionsgemisch nach Entfernung des Katalysators gegebenenfalls durch Erhitzen mit metallischem Natrium von organischen Chlorverbindungen gereinigt wird.

2. Verfahren nachh Anspruch 1,
dadurch gekennzeichnet,
daß als Katalysator das Aluminium-hydrosilikat-Mineral Montmorillonit eingesetzt wird.

3. Verfahren nach den Ansprüchen 1 und 2,
dadurch gekennzeichnet,
daß der Katalysator in einer Menge von 0,5 - 1 Gew.-%, bezogen auf Alkylbiphenyl der Formel II, eingesetzt wird.

4. Verfahren nach Anspruch 1,
dadurch gekennzeichnet,
daß Benzylchlorid in einer Menge von 0,5 Mol pro Mol Alkylbiphenyl der Formel II eingesetzt wird.

5. Verfahren nach Anspruch 1,
dadurch gekennzeichnet,
daß das Reaktionsgemisch nach Abtrennung des Katalysators mit 0,5 - 1 Gew.-% metallischem Natrium auf 200 - 220 °C zur Eliminierung organischer Chlorverbindungen erhitzt wird.

## Claims

1. A process for the preparation of monobenzylated alkylbiphenyls of the formula I, in which $R_1$ = methyl, $R_2$ = ethyl or isopropyl, $R_3$ = benzyl,

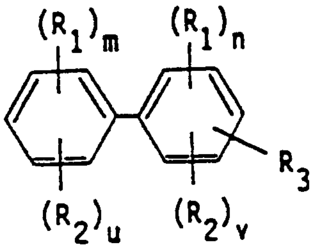

I

m, n, u and v = 0 - 2
$m + n \leqq 4$, $u + v \leqq 2$ and
$0 < (m + n) + (u + v) \leqq 4$
by reacting alkylbiphenyls of the formula II, in which $R_1$, $R_2$ and

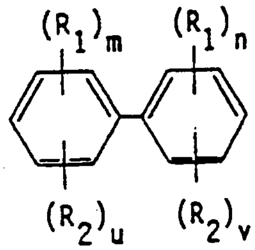

II

m, n, u, v have the same meanings as in formula I, with benzyl chloride,
characterized in that the reaction is carried out in the continuous-feed process using 0.2-0.7 mol of benzyl chloride per mole of alkylbiphenyl of the formula II in the presence of from 0.1 to 1.5% by weight, based on II, of a suspended aluminium hydrosilicate having the idealized formula $Al_2O_3 \bullet 4SiO_2 \bullet H_2O$ having a surface area of 190-250 $m^2/g$ at temperatures of from 100 to 140 °C, the continuous-feed period for the benzyl chloride extending over from 0.5 to 5 hours, and the reaction mixture, after removal of the catalyst, being purified if desired of organic chlorine compounds by heating with metallic sodium.

2. A process according to claim 1, characterized in that the catalyst used is the aluminium hydrosilicate mineral montmorillonite.

3. A process according to either of claims 1 and 2, characterized in that the catalyst is used in an amount of from 0.5 to 1% by weight, based on alkylbiphenyl of the formula II.

4. A process according to claim 1, characterized in that benzyl chloride is used in an amount of 0.5 mol per mole of alkylbiphenyl of the formula II.

5. A process according to claim 1, characterized in that the reaction mixture, after the catalyst has been separated off, is heated to 200-220 °C with 0.5-1% by weight of metallic sodium to eliminate organic chlorine compounds.

## Revendications

1. Procédé pour préparer des alkylbiphényles monobenzylés de formule I

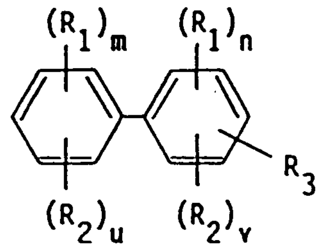

I

dans laquelle $R_1$ est le radical méthyle, $R_2$ est le radical éthyle ou isopropyle, $R_3$ est le radical benzyle,
m, n, u et v = 0-2,
$m + n \leqq 4$, $u + v \leqq 2$, et
$0 < (m + n) + (u + v) \leqq 4$
par réaction d'alkylbiphényles de formule II,

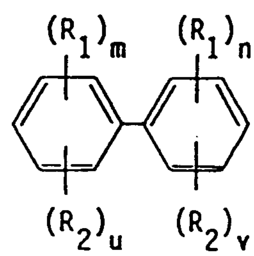

II

dans laquelle $R_1$, $R_2$ et les indices m, n, u et v ont les mêmes significations que dans la formule I, avec du chlorure de benzyle,
caractérisé en ce que la réaction est mise en oeuvre par introduction de 0,2 à 0,7 mole de chlorure de benzyle par mole d'alkylbiphényle de formule II, en présence d'une quantité de 0,1 à 1,5 % en poids, par rapport au composé de formule II, d'un hydrosilicate d'aluminium en suspension ayant la formule théorique $Al_2O_3.4SiO_2.H_2O$ ayant une aire spécifique de 190-250 $m^2/g$ à des températures de 100 à 140 °C, la durée de l'introduction du chlorure

de benzyle s'étendant sur un laps de temps de 0,5 à 5 heures, le mélange réactionnel, après élimination du catalyseur, étant éventuellement débarrassé des composés organiques du chlore par chauffage avec du sodium métallique.

**2.** Procédé selon la revendication 1, caractérisé en ce qu'on utilise comme catalyseur le produit minéral hydrosilicate d'aluminium qu'est la montmorillonite.

**3.** Procédé selon les revendications 1 et 2, caractérisé en ce qu'on utilise le catalyseur en une quantité de 0,5 à 1 % en poids par rapport à l'alkylbiphényle de formule II.

**4.** Procédé selon la revendication 1, caractérisé en ce qu'on utilise le chlorure de benzyle en une quantité de 0,5 mole par mole d'alkylbiphényle de formule II.

**5.** Procédé selon la revendication 1, caractérisé en ce que, après avoir séparé le catalyseur avec 0,5 à 1 % en poids de sodium métallique, on chauffe le mélange réactionnel à 200-220 °C pour éliminer les composés organiques du chlore.